# EUROPEAN PATENT SPECIFICATION

(11) **EP 1 962 694 B1**
(45) Date of publication and mention of the grant of the patent: **14.07.2010**
(21) Application number: 06846691.1
(22) Date of filing: 19.12.2006
(51) Int. Cl.: A61B 17/00, A61N 7/02

(54) **DEVICE FOR DETERMINING THE LOCATION OF A VASCULAR OPENING PRIOR TO APPLICATION OF HIFU ENERGY TO SEAL THE OPENING**
VORRICHTUNG ZUR BESTIMMUNG DER POSITION EINER GEFÄSSÖFFNUNG VOR DER ANWENDUNG VON HIFU-ENERGIE ZUR VERSIEGELUNG DER ÖFFNUNG
DISPOSITIF POUR DETERMINER L'EMPLACEMENT D'UNE OUVERTURE VASCULAIRE PREALABLEMENT A L'APPLICATION D'UNE ENERGIE HIFU POUR FERMER L'OUVERTURE

(30) Priority: 22.12.2005 US 316059
(43) Date of publication of application: 03.09.2008
(73) Proprietor: Boston Scientific Scimed, Inc., Maple Grove, MN 55311-1566 (US)
(72) Inventor: WILLIS, Parker, N., Atherton, CA 94027 (US)
(74) Representative: Cabinet Plasseraud
(86) International application number: PCT/US2006/062310
(87) International publication number: WO 2007/073551

(56) References cited:
- EP-A- 1 449 563
- WO-A-99/58055
- US-A1- 2005 240 170
- US-B1- 6 511 428
- US-B1- 6 656 136

## Description

### TECHNICAL FIELD

Apparatuses for sealing vascular punctures and openings are disclosed. More specifically, systems are disclosed to determine an accurate location of the vascular opening being treated so that energy delivered by a high intensity focused ultrasound (HIFU) device is accurately delivered to the opening with minimal damage to surrounding tissue and discomfort for the patient.

### BACKGROUND OF THE RELATED ART

Various surgical procedures are performed by medical specialists such as cardiologists and radiologists, utilizing percutaneous entry into a blood vessel. Io facilitate cardiovascular procedures, a small gauge needle is introduced through the skin and into a target blood vessel, often the femoral artery. The needle forms a puncture through the blood vessel wall at the distal end of a tract that extends through the overlying tissue. A guide wire is then introduced through the bore of the needle, and the needle is withdrawn over the guide wire. An introducer sheath is next advanced over the guide wire. The sheath and guide wire are left in place to provide access during subsequent procedures.

The sheath facilitates passage of a variety of diagnostic and therapeutic instruments and devices into the vessel and its tributaries. Such diagnostic procedures may include angiography, intravascular ultrasonic imaging, and the like. Typical interventional procedures include angioplasty, atherectomy, stent and graft placement, embolization, and the like. After a procedure is completed, the catheters, guide wite, and introducer sheath are removed, and it is necessary to close the vascular puncture to provide hemostasis and allow healing.

The common technique for achieving hemostasis is to apply pressure, either manually or mechanically, on the patient's body in the region of the tissue tract and vascular puncture. Initially, pressure is applied manually and subsequently is maintained through the use of mechanical clamps and other pressure-applying devices. While effective in most cases, the application of external pressure to the patient's skin presents a number of disadvantages. For example, when applied manually, the procedure is time-consuming and requires the presence of a medical professional for thirty minutes or more. For both manual and mechanical pressure application, the procedure is uncomfortable for the patient and frequently requires the administration of analgesics to be tolerable.

Moreover, complications from manual pressure application are common. The application of excessive pressure can occlude the underling artery, resulting in ischemia and/or thrombosis. Even after hemostasis has apparently been achieved, the patient must remain immobile and under observation for hours to prevent dislodgment of the clot and to assure that bleeding from the puncture wound does not resume. Renewed bleeding through the tissue tract is not uncommon which can result in hematoma, pseudoaneurisms, and arteriovenous fistulas. Such complications may require blood transfusion, surgical intervention, or other corrective procedures. The risk of these complications increases with the use of larger sheath sizes, which are frequently necessary in interventional procedures, and when the patient is anticoagulated with heparin or other drugs.

As a result, several alternatives to the manual pressure hemostasis technique have been proposed to address the problem of sealing vessel wall opening following percutaneous transcatheter procedures. For example, bioabsorbable, thrombogenic plugs comprising collagen and other materials have been used at the vessel wall opening to stop bleeding. These large hemostasis plugs stimulate blood coagulation at the vessel opening, but they block the catheter entry tract, making catheter reentry difficult, if needed. Other techniques provide for the use of small dissolvable disks or anchors that are placed in the vessel to block or clamp the opening. However, these devices are problematic as any device retaining in the vessel lumen increases the risk of thrombus formation. Such devices also can detach and cause occlusion in a distal section of the blood vessel, which would need to be surgically removed.

Additional techniques use needles and sutures delivered through catheters are used to ligate the opening. Obviously, this suturing procedure requires a high level of skill and suture material left in the vessel may cause tissue irritation that will prolong the healing process. Another technique involves the injection of a procoagulant into the opening with a balloon catheter blocking inside the vessel lumen. However, it is possible for the clotting agent to leak past the balloon into the vessel lumen and cause stenosis. Lasers and radio-frequency (RF) energy have also been used to thermally fuse or weld the punctured tissue together. Other mote recent techniques involve the use of high frequency ultrasound (HIFU) energy.

HIFU seals vascular openings by quickly increasing the temperature of surrounding tissue by 70-90°C in a matter of seconds. The temperature rise mobilizes and denatures collagen, forming coagulum, which rapidly seals the opening.

However, alternatives for accurately delivering a HIFU dose is desirable. Two examples can be found in U.S Patent No. 6,656,136 and U.S. Patent Application Publication Nos. 2004/0106880 and 2005/0080334. Document US-A-6, 511, 428 discloses an ultrasound therapy apparatus comprising an urethral catheter with an ultrasound transducer acting as an emitter and an intrarectal applicator housing a HIFU array and an imaging array acting as a receiver for locating in two dimensions the position of the transducer of the urethral catheter. The apparatuses disclosed herein are further contributions to the art of accurately navigating and dosing HIFU energy at a vessel opening to seal the opening with minimal damage to surrounding tissue or discomfort for the patient.

### SUMMARY OF THE DISCLOSURE

In satisfaction of the aforenoted needs, a medical system is disclosed that comprises a probe comprising a distal end, at least one transducer mounted on the distal end of the probe, and a high intensity focused ultrasound (HIFU) device comprising a therapeutic array, an imaging array, at least three transducers mounted around the therapeutic array, and related processing circuitry. The transducers on the probe and on the HIFU device generate acoustic energy that passes through the tissue, The acoustic energy is converted to electrical signals by transceiver circuitry, and the electrical signals are used to produce time-of-flight (TOF) measurements, which, in turn, are used to generate a three-dimensional position of the end of the probe with respect to the HIFU device and the therapeutic array of the HIFU device.

The transducer(s) on the end of the probe is preferably used as a transmitter and the transducers mounted on the HIFU device are preferably used as receivers so that multiple I OF measurements may be simultaneously made for each transducer on the HIFU device vis à vis the end of the probe. The three dimensional position of the end of the probe may be calculated from the I OF measurement data using a multi-dimensional scaling (MDS) algorithm or other suitable algorithm that will be apparent to those skilled in the art. The processing circuitry may also calculate local coordinates of the distal end of the probe relative to the imaging and therapeutic arrays using a Procrustean similarity transform (PSI) or other suitable algorithm that will be apparent to those skilled in the art.

Using the imaging array and the acoustic data generated from the use of the transducers, the distal end of the probe can be guided into the vessel opening and the focus point of the HIFU device can be adjusted to match the three-dimensional position and the coordinates of the distal end of the probe so the operator is assured that the HIFU focus point is accurate and not out of plane. With currently available technology, independent confirmation that the plane of the HIFU focus matches that of the vessel opening is not possible.

In a refinement, the transducer at the end of the probe is a pressure transducer as well as an acoustic transducer or the probe tip may include separate acoustic and pressure transducers. In a related refinement, one of the transducer at the end of the probe is polyvinylidene difluoride (PVDF) piezo electric film transducer, a polyvinylidene fluoride (PVF) piezo electric transducer or a piezo electric copolymer transducer. Copolymer piezo electric transducers are often copolymers of vinylidene fluoride, but the use of other copolymers are possible. In such an embodiment, the use of an imaging array may be eliminated or rendered superfluous as the vessel opening may be located using the pressure sensing capabilities of the probe tip In short, a pressure change as the probe tip enters the vessel opening is detected.

In another refinement, the at least three reference transducers fixedly mounted around the therapeutic array comprise four transducers. For more efficient IOF calculations, these four transducers are used as receivers and the at least one transducer at the probe tip is used as a transmitter. In a related refinement, the transducers may be of the ceramic type, the PVDF type or the piezo electric copolymer type.

In another refinement, the MDS performed by the control circuitry or software is performed multiple times for different sets of three transducers mounted around the HIFU device. In the event a large discrepancy is generated due to a noisy or defective transducer, use of that noisy or defective transducer in future calculations can be stopped. In related refinement, the results of the MDS calculations are averaged.

In another refinement, the controller, processor or control circuitry or software calculates a focal depth for HIFU therapy based on the PST or use of a similar algorithm.

In another refinement, the device generates an alarm if the therapeutic array is displaced laterally or longitudinally from the distal end of the probe by a distance greater than a predetermined threshold distance.

Other advantages and features of the disclosed embodiments will be best understood upon reference to the accompanying drawings and detailed description that follows.

### BRIEF DESCRIPTION OF THE DRAWINGS

The disclosed systems will be described more or less diagrammatically in the accompanying drawings, wherein:

Fig. 1 is a sectional view of a portion of a body, illustrating a catheter and guide wire extending through a puncture that extends transdermally into an artery or vessel;

Fig. 2 is another sectional view the body as shown in Fig. 1, illustrating the vessel and skin openings or puncture sites;

Fig. 3 is a cross-sectional view of a portion of the body and an end view of a HIFU applicator, schematically the application of HIFU energy to a puncture in an artery or vessel;

Fig. 4 is a bottom plan view of the applicator shown in Fig. 3 particularly illustrating two therapeutic arrays and an imaging array disposed therebetween and four reference transducers mounted at fixed positions around the therapeutic arrays;

Fig. 5 is a plan view of a probe adapted to be inserted into a puncture wound over a guide wire and that is equipped with a plurality of acoustic transducers at a distal end thereof;

Fig. 6 is a cross-sectional view of a portion of a body like that shown in Fig. 1 and 2, illustrating the probe of Fig. 5 in use to determine a location of an opening or puncture in the vessel the applicator disposed above the puncture site and resting on the skin of the patient; and

Fig. 7 is a flow diagram schematically illustrating the various calculations and process circuitry of the disclosed HIFU device for locating vessel openings.

It should be understood that the drawings are not necessarily to scale and that the disclosed embodiments are illustrated using diagrammatic representations and fragmentary views. In certain instances, details may have been omitted which are not necessary for an understanding of the disclosed embodiments or which render other details difficult to perceive. It should be understood, of course, that the sample shaking apparatus is not necessarily limited to the particular embodiments disclosed herein.

### DETAILED DESCRIPTION OF THE PRESENTLY PREFERRED EMBODIMENTS

Ultrasound can be brought into a tight focus at relatively short distances from the source or origin. If ultrasound with a sufficient energy is radiated into animal tissue, cells located within the focal volume or plane or rapidly heated while surrounding tissues are unaffected. The surrounding tissues are unaffected primarily because ultrasound energy outside of the focal volume is of' a low density and therefore the heating of the surrounding tissues is minimal and low impact.

High-intensity focused ultrasound (HIFU) applications utilize ultrasound intensities in excess of 1,000 watts/cm². At a focal point, these intensities can result in large, controlled temperature rises within a matter of seconds. These large, controller temperature rises are useful in sealing holes or punctures in blood vessels. Specifically, when accurately targeted on a vascular opening, HIFU has been shown to induce hemostasis in less a minute. In addition to blood vessel punctures, HIFU can be used in the treatment of other lacerations as well.

The mechanism of the hemostasis induced by HIFU involves the denaturization of perivascular collagen, which results in the formation of coagulum and/or fibron that seals the hole or puncture HIFU has been observed to play an effective role in sealing vessel punctures of varying sizes.

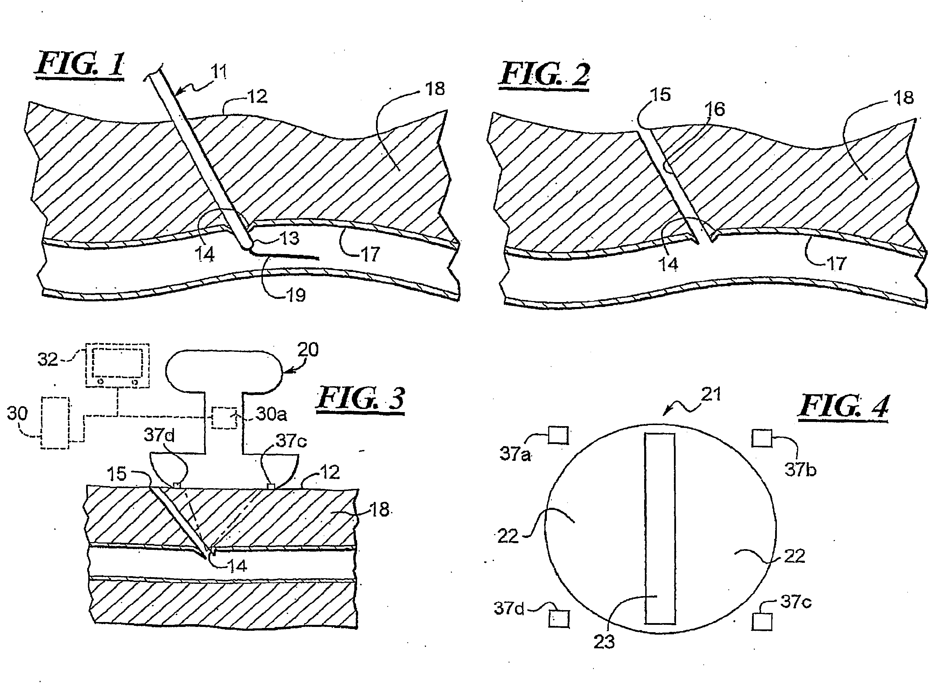

Turning to Fig. 1, a catheter 11 has been inserted through a patient's skin 12 and a distal end 13 of the catheter through a vessel puncture site 14. The vessel puncture site 14 and skin puncture site 15 are better shown in Fig 2. The channel 16 left by the catheter 11 and other related devices obviously presents a serious bleeding problem, particularly when the vessel 17 is a major artery, such as the femoral artery. Various epidermal and other tissue layers are indicated generally at 18.

Turning to Fig. 3, a typical HIFU applicator 20 is shown as applied to a patient's skin 12. The applicator 20 includes a lower surface 21 that, as shown in Fig. 4, includes two separate arrays, including therapeutic arrays 22 separated by an imaging array 23. Essentially, the applicator 20 is an annular phase array ultrasound transducer with depth focusing. The image array 23 is used to transmit data to a separate controller 30 or processing circuitry 30a disposed within the device 20 (or a combination thereof) which translates the data and generates visual images on a monitor 32. The circuitry 30, 30a and the related software may be distributed among the housing of the device 20 and a housing for the controller 30. The operator watches the monitor 32 to ensure that the applicator 20 is disposed on the outer skin in general lateral and longitudinal proximity to the vessel puncture 14.

The clinical challenge is to position the applicator 20 over the vessel puncture 14 and apply the HIFU energy at the correct focal depth to seal the puncture 14 without damaging the surrounding tissue 18. This is clinically challenging because the vessel puncture site 14 is distinct from the skin puncture site 15. Thus, the lateral and longitudinal alignment of the applicator 20 with the vessel puncture site 14 is not straightforward.

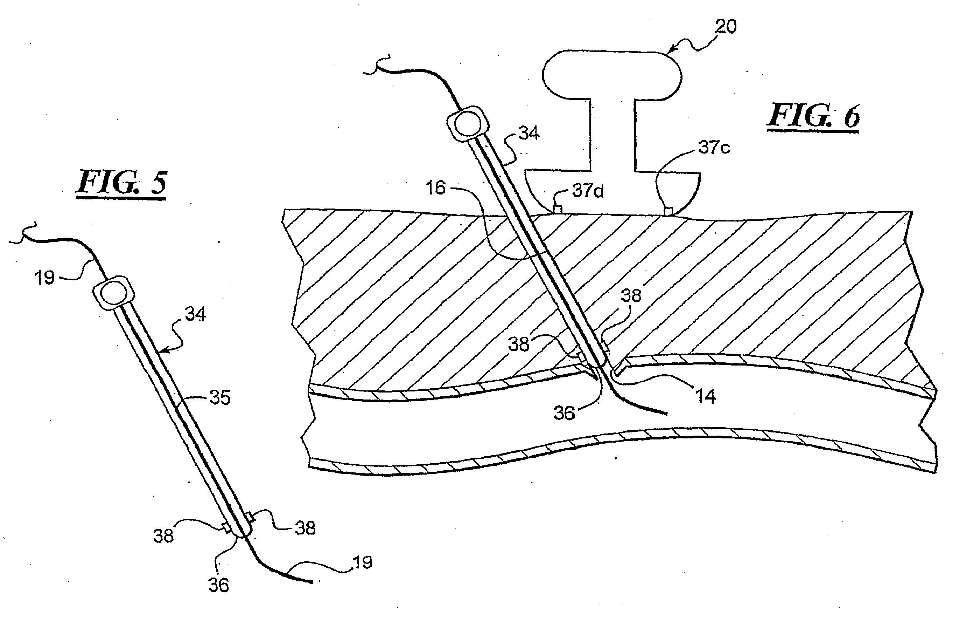

Theoretically, the image array 23 can be used to guide the device over the vessel puncture site 14. However, the interpretation of exactly where the vessel punctuate site 14 is relative to the applicator 20 can become complicated. Therefore, use of a probe 34 that can be slid over a guidewire 19 has proven helpful. The probe 34, as shown in Fig_{.} 5, includes an outer sheath 35 with a distal end 3 6 that includes one or more acoustic transducers 38 mounted to the distal end 36 of the probe 34. To assist in proper positioning, transducers 37a-37d are fixed around the therapeutic array 22 of the applicator 20 as shown in Figs. 4 and 6 and are used with the transducer 38 on the probe 34 as discussed in greater detail below.

As shown in Fig. 6, the transducers 37a-37d and 38 generate and receive acoustic energy that passes through the patient's tissue 18. Preferably, the probe transducer 38 acts as a transmitter and the applicator transducers 37a-37d act as receivers or reference transducers but a reverse orientation is possible. It is preferred that the probe transducer 38 acts as a transmitter and the applicator transducers 37a-3 7d act as receivers because in this orientation the circuitry can process multiple time-of-flight (TOF) calculations simultaneously.

Still referring to Fig. 6, the probe 34 is inserted into the channel 16 and is pushed down the channel 16 until the distal end 36 of the probe 34 is disposed at the vessel opening 14. During this process, the operator may use the imaging array 23 in combination with the local coordinate calculations that are based on the TOF measurements made possible by the one or more probe transducers 38 and the three or more applicator transducers 37a-37d. Use of the imaging array 23 and the images displayed on the video screen 32 enables the operator to watch the screen 32 as he/she manipulates the distal end 36 of the probe 34 to the desired location at or in the vessel opening 14. After the desired position is achieved, through use of the imaging array 23 of the applicator 20, the position of the probe 34 is held and the location of the distal end 36 of the probe 34 is determined by the acoustic energy exchanged by transducers 37, 38. as interpreted by the controlled 30 or control circuitry 30a.

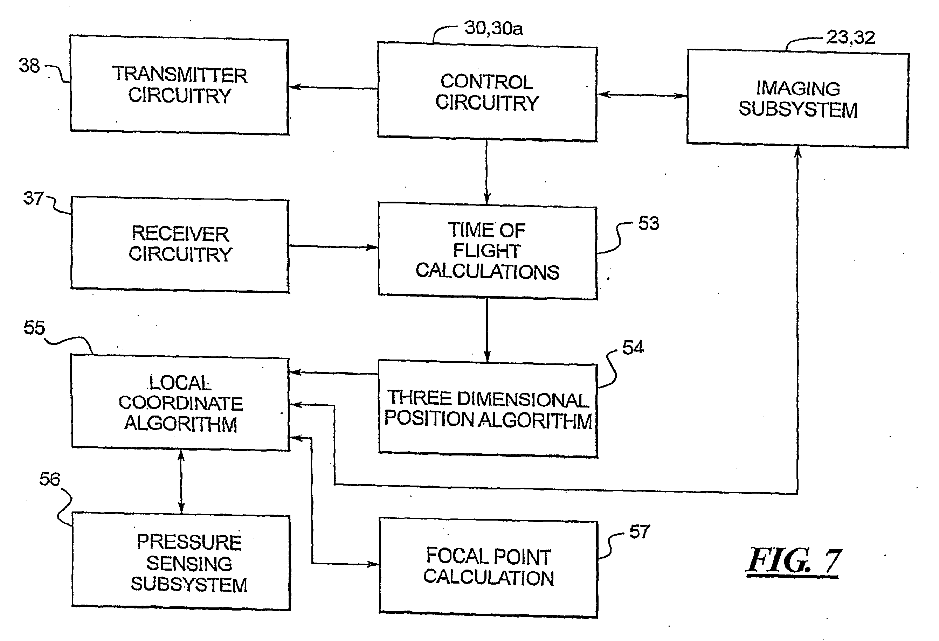

In summary, referring to Fig. 7, as the receiver transducers 37 receive acoustic energy created by the probe transducer 38, electrical signals are generated and transmitted to the transceiver circuitry at 52 which may reside in the applicator 20 (see 30a) or in a separate controller 30 or a combination of the two. The transceiver circuitry performs TOF calculations at 53 to determine distances between the probe transducer 38 and any one of the applicator transducers 37a-37d. The control circuitry 30, 30a controls the timing of the transducer(s) 38 and is in communication with the imaging array subsystem 23, 32. The TOF measurements are used to generate three-dimensional position data at 54, which in turn is used to generate local coordinates at 55. The local coordinate calculations are used in combination with video images on the screen 32 or with pressure change signals generated at 56 to make sure that the probe tip 36 is at the vessel opening and not out of plane. Using a "finial" local coordinate calculation, or the calculation performed by the circuitry or software after the operator is sure the probe tip 36 is at the vessel opening, a focal point calculation is performed at 57. Again, use of a pressure sensing transducer at the probe tip 36 may prove to be simpler and more straightforward that use of an imaging array 23.

If an imaging array 23 and video display 32 is used, the video data should be transmitted at a different frequency than acoustic energy transmitted by the transducers 38 or 37 to prevent acoustic crosstalk or interference between the TOF and imaging measurements.

A preferred method of generating the three-dimensional position data involves the use of a MDS algorithm as explained below. The local coordinates of the distal end 36 of the probe 34 are calculated at 55, preferably using a PSI algorithm. The local coordinates are used in conjunction with image array data to confirm the location of the probe tip 36 with respect to the vessel opening 14 (see 56 in Fig. 7) and to confirm that the focal point determination at 57 is in the correct plane. The above process is repeated every time the applicator 20 and/or probe 34 is moved or manipulated as the operator moves the probe tip 36 into the position shown in Fig. 6 and the applicator 20 to a position where the focal plane of the therapeutic arrays is coplanar with the vessel opening 14.

The term "control circuitry" is intended to cover the software, circuitry, programmable memory, processor, microprocessor chips, boards or other processing means indicated schematically at 30, 30a in the drawings that (1) convert the signals from the transducers into TOF calculations, that (2) convert the TOF calculations into three-dimensional position data and that (3) convert the three-dimensional position data into local coordinates. The location of the devices and software that perform these functions is unimportant. They may reside in the applicator 20 housing itself, in a separate controller box or panel 30 or a combination thereof.

More than one transducer 38 on the distal end 36 of the probe 34 can be utilized. The imaging array 23 could be eliminated or made redundant if the transducer 38 includes a pressure sensing capability or if a separate pressure sensing transducer was added to the probe end. A transducer 38 made from polyvinylidene difluoride (PVDF), polyvinylidene fluoride (PVF), PVDF piezo electric, PVF piezo electric or piezo electric copolymer materials can be utilized. PVD film, PVDF film, PVF piezo electric film, PVDF piezo electric film, copolymer film and piezo electric copolymer film transducers are known in the art. U.S. Patent Nos 6,835,178, 6,504,289, 6485432, 6,387,051, and 6217,518 disclose such pressure sensing transducers. Instead of relying on the imaging array, the pressure transducer can be used to determine when the probe top 36 enters the opening 14 of the vessel.

the example shown, four transducers 37a-37d are mounted directly on the applicator 20 as indicated in Figs. 3 and 4. Since the transducers 37a-37d are mechanically fixed to the applicator 20, it is not necessary to measure acoustically the distance between the transducers 37a-37d. The distance is controlled at the time of manufacture and is therefore known.

Only three transducers 3 7 are required on the HIFU applicator 20. The use of four applicators 37 in this disclosure is an exemplary embodiment and allows for redundant calculations to increase the confidence of the calculated three-dimensional position of the distal end 36 of the probe 34 or the transducer 38 on the probe 34 and provide for the possibility of rejection of noisy measurements or in the event one or more of the transducers 37a-37d malfunctions.

The control circuitry 30, 30a utilizes a basic algorithm for determining if the three-dimensional position of the distal end 36 of the probe 34 with respect to the applicator 20 That basic algorithm may be a multi-dimensional scaling (MDS) algorithm. Given N reference transducers 37 mounted on the applicator 20 and one transducer 38 mounted on the probe tip 36, the input to the algorithm is aN + 1 x N + I matrix of input distances as follows where D_{i,j} = the distance between the ith and jth transducer and D_{i,j} = 0 because the distance of a transducer to itself is 0ₐ It will be noted that only the last column and row of the matrix is actually calculated as the N x N sub matrix is determined by the mount location of the reference transducers 37.

The MDS algorithm provides an output matrix P of positions, ie, D_{N+1xN+1} → P_{3xN+1} wherein in each column of P is the x, y, z coordinates of a single transducer 37 position. In general, the output positions P have an arbitrary translation rotation and reflection. That is, there are an infinite number of output positions that match the input distances D. The positions P can be transformed to a local coordinate system of the applicator 20 using the procrustean similarity transform (PST) or other suitable algorithm. This algorithm matches the position of the two point sets from two different coordinate systems in a L₂ norm sense. In this case, one would match the output positions of the N preference transducers 37 as provided by the MDS output coordinate system with the local coordinate system of the applicator 20. This would follow that registration of the applicator device 20 coordinates for both therapy and imaging with the three-dimensional position of the distal end 36 of the probe 34.

Thus, using three or more fixed transducers 37 mounted on the applicator 20 and at least one transducer 38 disposed at the distal end 36 of the probe 34, a more accurate distance calculation between the arrays 22 of the applicator 20 and the distal end of the probe 36 and therefore the puncture site 14 are provided. Therefore, a more accurate focal volume for the therapeutic arrays 22 can be calculated and the HIFU energy can be more accurately applied with minimal damage to surrounding tissue and organs.

Utilizing four or more reference transducers 37a-37d can provide a convenient redundancy. Specifically, because only three reference transducers are needed for an accurate calculation, the controller can perform the distance calculation multiple times and the results using a noisy or defective transducer can be ignored. Thus, the operator is assured that the calculation is accurate and not affected by transducer malfunction or noise.

While only certain embodiments have been set forth, alternative embodiments and various modifications will be apparent from the above description to those skilled in the art. These and other alternatives are considered within the scope of this disclosure.

## Claims

1. A medical system comprising:
a probe (34) comprising a distal end (36);
at least one probe transducer (38) mounted on the distal end (36) of the probe (34);
a high intensity focused ultrasound (HIFU) device (20) comprising a therapeutic array (22), and control circuitry (30), the HIFU device (20) further comprising at least three applicator transducers (37a-d) mounted around the therapeutic array (22);
one of the probe and applicator transducers (38, 37a-d) adapted to generate acoustic energy and the other of the probe and applicator transducers (38, 37a-d) adapted to receive said acoustic energy and generate electrical signals in response thereto;
the control circuit (30) adapted to perfom time-of-flight (TOF) calculations based on the electrical signals that indicate distances between the probe transducer (38) and individual applicator transducers (37a-d), the control circuitry adapted to perform calculations generating three-dimensional position data of the probo transducer (38) with respect to the applicator transducers (37a-d) and the therapeutic array (22) based on the TOF calculations, and the control circuitry adapted to perform local coordinate calculations for the probe transducer (38) with respect to the therapeutic array (22) based on the three-dimensional position data.

2. The medical system of claim 1 wherein the control circuitry (30) is adapted to calculate the three-dimensional position data with a multi-dimensional scaling (MDS) algorithm.

3. The medical system of claim 2 wherein the control circuitry (30) is adapted to perform the local coordinate calculations using a Procrustean similarity transform (PST).

4. The system of claim 1, wherein the at least three applicator transducers (37a-d) comprise four transducers.

5. The system of claim 4 wherein the three-dimensional position data calculations performed by the control circuitry are performed a plurality of times for different sets of three of the four applicator transducers.

6. The system of claim 5 wherein the results of the three-dimensional position data calculations are averaged.

7. The system of claim 1 further comprising a pressure transducer disposed at the probe's distal end (36).

8. The system of claim 1 wherein the probe transducer (38) is a combined acoustic and pressure transducer.

9. The system of claim 1 wherein the controller (30) is adapted to calculate a focal depth for HIPU therapy based on the local coordinate calcutation.

10. The system of claim 1 wherein the controller (30) is adapted to generate an alarm if the therapeutic array (22) is displaced laterally or longitudinally from the distal end (36) of the probe (34) by a distance greater than a predetermined threshold distance.

11. A high intensity focused ultrasound (HIFU) therapy system, comprising:
a probe (34) comprising a distal end (36);
at least one acoustic transducer (38) mounted on the distal end (36) of the probe (34);
an applicator (20) comprising a therapeutic array (22) and an imaging array (23), the applicator (20) being linked to a control circuitry (30), the applicator (20) further comprising at least three reference transducers (37a-d) mounted around the therapeutic array (22);
the probe transducer (38) adapted to transmit acoustic energy to the reference transducer (37a-d), the reference transducers (37a-d) adapted to generate electrical signals as a result of the acoustic energy received from the probe transducer (38);
the imaging array (23) adapted to generate and transmit signals indicative of locations of the probe (34) and surrounding tissue structures;
the control circuitry (30) adapted to perform time-of-flight (TOF) calculations based on the electrical signals that indicate distances between the probe transducer (38) and individual reference traansducers (37a-d), the control circuitry (30) adapted to generate three-dimensional position data of the probe transducer (38) with respect to the reference transducers (37a-d) and the therapeutic array (22) based on the TOF calculations, and the control circuitry (30) adapted to generate local coordinate information of the probe transducer (38) with respect to the therapeutic array (22).

12. The HIFU system of claim 11 wherein the control circuitry (30) is adapted to generate the three-dimensional position data with a multi-dimensional scaling (MDS) algorithm.

13. The HIFU system of claim 12 wherein the control circuitry (30) is adapted to generate the local coordinate informations using a Procrustean similarity transform (PST).

14. The HIFU system of claim 11 further comprising a pressure transducer disposed at the probe's distal end (36).

15. The HIFU system of claim 11 wherein the probe transducer (38) is a combined acoustic and pressure transducer.

## Patentansprüche

1. Medizinisches System, mit:
einer Sonde (34) mit einem distalen Ende (36);
zumindest einem Sondenwandler (38), der am distalen Ende (36) der Sonde (34) montiert ist;
eine hochintensiver-fokussierter-Ultraschall-(HIFU)-Einrichtung (20) mit einem therapeutischen Bereich (22) und einem Steuerschaltkreis (30), wobei die HIFU-Einrichtung (20) weiterhin zumindest drei um den therapeutischen Bereich (22) montierte Applikatorwandler (37a-d) aufweist;
wobei einer der Sonden- und Applikatorwandler (38, 37a-d) dazu ausgebildet ist, akustische Energie zu erzeugen, und wobei die anderen der Sonden- und Applikatorwandler (38, 37a-d) dazu ausgebildet sind, jene akustische Energie zu empfangen und als Antwort darauf elektrische Signale zu erzeugen;
wobei der Steuerschaltkreis (30) dazu ausgebildet ist, Laufzeitberechnungen (TOF) basierend auf den elektrischen Signalen, die Abstände zwischen dem Sondenwandler (38) und den einzelnen Applikatorwandlern (37a-d) anzeigen, durchzuführen, wobei der Steuerschaltkreis dazu ausgebildet ist, Berechnungen durchzuführen, die dreidimensionale Positionsdaten des Sondenwandlers (38) bezüglich der Applikatorwandler (37a-d) und des therapeutischen Bereichs (22) basierend auf den TOF-Berechnungen erzeugen, wobei der Steuerschaltkreis dazu ausgebildet ist, lokale Koordinatenberechnungen für den Sondenwandler (38) bezüglich des therapeutischen Bereichs (22) basierend auf den dreidimensionalen Positionsdaten durchzuführen.

2. Medizinisches System nach Anspruch 1, wobei der Steuerschaltkreis (30) dazu ausgebildet ist, die dreidimensionalen Positionsdaten mit einem multidimensionalen Skalierungsalgorithmus (MDS) zu berechnen.

3. Medizinisches System nach Anspruch 2, wobei der Steuerschaltkreis (30) dazu ausgebildet ist, die lokale Koordinatenberechnung unter Verwendung einer Procrustes-Ähnlichkeitstransformation (PST) durchzuführen.

4. System nach Anspruch 1, wobei die mindestens drei Applikatorwandler (37a-d) vier Wandler aufweisen.

5. System nach Anspruch 4, wobei die durch den Steuerschaltkreis durchgeführten dreidimensionalen Positionsdatenberechnungen mehrfach für verschiedene Gruppen mit jeweils drei der vier Applikatorwandler durchgeführt werden.

6. System nach Anspruch 5, wobei die Ergebnisse der dreidimensionalen Positionsdatenberechnungen gemittelt werden.

7. System nach Anspruch 1, weiterhin aufweisend einen am distalen Ende (36) der Sonde angeordneten Druckwandler.

8. System nach Anspruch 1, wobei der Sondenwandler (38) ein kombinierter Akustik-und Druckwandler ist.

9. System nach Anspruch 1, wobei die Steuereinheit (30) dazu ausgebildet ist, eine fokale Tiefe für die HIFU-Therapie basierend auf den lokalen Koordinatenberechnungen zu berechnen.

10. System nach Anspruch 1, wobei die Steuereinheit (30) dazu ausgebildet ist, einen Alarm zu erzeugen, wenn der therapeutische Bereich (42) bilateral oder longitudinal von dem distalen Ende (36) der Sonde (34) um einen Abstand verlagert wird, der größer ist als ein vorbestimmter Schwellenabstand.

11. Hochintensiver-fokussierter-Ultraschall-(HIFU)-Therapiesystem, mit:
einer Sonde (34) mit einem distalen Ende (36);
zumindest einem Akustikwandler (38), der am distalen Ende (36) der Sonde (34) montiert ist;
einem Applikator (20) mit einem therapeutischen Bereich (22) und einem abbildenden Bereich (23), wobei der Applikator (20) mit einem Steuerschaltkreis (30) verbunden ist, wobei der Applikator (20) weiterhin zumindest drei Referenzwandler (37a-d) aufweist, die um den therapeutischen Bereich (22) montiert sind;
wobei der Sondenwandler (38) dazu ausgebildet ist, akustische Energie zu den Referenzwandlern (37a-d) zu übertragen, wobei die Referenzwandler (37a-d) dazu ausgebildet sind, als Antwort auf die vom Sondenwandler (38) empfangene akustische Energie elektrische Signale zu erzeugen;
wobei der abbildende Bereich (23) dazu ausgebildet ist, Signale zu erzeugen und zu übertragen, die indikativ für die Orte der Sonde (34) und umgebende Gewebestrukturen sind;
wobei der Steuerschaltkreis (30) dazu ausgebildet ist, Laufzeitberechnungen (TOF) basierend auf den elektrischen Signalen, die Abstände zwischen dem Sondenwandler (38) und den einzelnen Referenzwandlern (37a-d) anzeigen, durchzuführen, wobei der Steuerschaltkreis (30) dazu ausgebildet ist, dreidimensionale Positionsdaten des Sondenwandlers (38) bezüglich der Referenzwandler (37a-d) und des therapeutischen Bereichs (22) basierend auf den TOF-Berechnungen zu erzeugen, und wobei der Steuerschaltkreis (30) dazu ausgebildet ist, lokale Koordinateninformationen des Sondenwandlers (38) bezüglich des therapeutischen Bereichs (22) zu erzeugen.

12. HIFU-System nach Anspruch 11, wobei der Steuerschaltkreis (30) dazu ausgebildet ist, die dreidimensionalen Positionsdaten mit einem multidimensionalen Skalierungsalgorithmus (MDS) zu erzeugen.

13. HIFU-System nach Anspruch 12, wobei der Steuerschaltkreis (30) dazu ausgebildet ist, die lokalen Koordinateninformationen unter Verwendung einer Procrustes-Ähnlichkeitstransformation (PST) zu erzeugen.

14. HIFU-System nach Anspruch 11, weiterhin aufweisend einen am distalen Ende (36) der Sonde angeordneten Druckwandler.

15. HIFU-System nach Anspruch 11, wobei der Sondenwandler (38) ein kombinierter Akustik-und Druckwandler ist.

## Revendications

1. Système médical comprenant :
une sonde (34) comprenant une extrémité distale (36) ;
au moins un transducteur de sonde (38) monté sur l'extrémité distale (36) de la sonde (34) ;
un dispositif à ultrasons focalisé à haute intensité (HIFU) (20) comprenant un agencement thérapeutique (22) et un circuit de commande (30), le dispositif HIFU (20) comprenant en outre au moins trois transducteurs d'applicateur (37a-d) montés autour de l'agencement thérapeutique (22) ;
un élément parmi les au moins un transducteurs de sonde et les transducteurs d'applicateur (38, 37a-d) étant adapté pour générer une énergie acoustique et l'autre élément parmi les au moins un transducteurs de sonde et les transducteurs d'applicateur (38, 37a-d) étant adapté pour recevoir ladite énergie acoustique et générer des signaux électriques en réponse à celle-ci ;
le circuit de commande (30) étant adapté pour réaliser des calculs de temps de vol (TOF) basés sur les signaux électriques qui indiquent des distances entre le transducteur de sonde (38) et des transducteurs d'applicateur individuels (37a-d), le circuit de commande étant adapté pour réaliser les calculs générant des données de position en trois dimensions du transducteur de sonde (38), relativement aux transducteurs d'applicateur (37a-d) et l'agencement thérapeutique (22), sur la base des calculs TOF, et le circuit de commande étant adapté pour réaliser des calculs de coordonnées locales pour le transducteur de sonde (38) relativement à l'agencement thérapeutique (22), sur la base des données de position en trois dimensions.

2. Système médical selon la revendication 1, dans lequel le circuit de commande (30) est adapté pour calculer les données de position en trois dimensions avec un algorithme d'échelle multidimensionnelle (MDS).

3. Système médical selon la revendication 2, dans lequel le circuit de commande (30) est adapté pour effectuer les calculs de coordonnées locales en utilisant une transformation de similarité Procrustéenne (PST).

4. Système selon la revendication 1, dans lequel lesdits au moins trois transducteurs d'applicateur (37a-d) comprennent quatre transducteurs.

5. Système selon la revendication 4, dans lequel les calculs de données de position en trois dimensions effectués par le circuit de commande sont réalisés plusieurs fois pour différents ensembles de trois des quatre transducteurs d'applicateur.

6. Système selon la revendication 5, dans lequel les résultats des calculs de données de position en trois dimensions sont donnés en moyenne.

7. Système selon la revendication 1, comprenant en outre un transducteur de pression disposé à l'extrémité distale de la sonde (36).

8. Système selon la revendication 1, dans lequel le transducteur de sonde (38) est un transducteur de pression et acoustique combiné.

9. Système selon la revendication 1, dans lequel le contrôleur (30) est adapté pour calculer une profondeur focale pour le traitement par HIFU, sur la base du calcul de coordonnées locales.

10. Système selon la revendication 1, dans lequel le contrôleur (30) est adapté pour générer une alarme si l'agencement thérapeutique (22) est déplacé latéralement ou longitudinalement de l'extrémité distale (36) de la sonde (34) sur une distance supérieure à une distance seuil prédéterminée.

11. Système de traitement thérapeutique à ultrasons focalisé haute intensité (HIFU) comprenant :
une sonde (34) comprenant une extrémité distale (36) ;
au moins un transducteur de sonde (38) monté sur l'extrémité distale (36) de la sonde (34) ;
un applicateur (20) comprenant un agencement thérapeutique (22) et un agencement d'imagerie (23), l'applicateur (20) étant lié à un circuit de commande (30), l'applicateur (20) comprenant en outre au moins trois transducteurs de référence (37a-d) montés autour de l'agencement thérapeutique (22) ;
le transducteur de sonde (38) étant adapté pour transmettre de l'énergie acoustique aux transducteurs de référence (37a-d), les transducteurs de référence (37a-d) étant adaptés pour générer des signaux électriques du fait de l'énergie acoustique reçue du transducteur de sonde (38) ;
l'agencement d'imagerie (23) étant adapté pour générer et transmettre des signaux indicatifs des emplacements de la sonde (34) et entourant les structures de tissu ;
le circuit de commande (30) étant adapté pour effectuer des calculs de temps de vol (TOF) basés sur les signaux électriques, qui indiquent les distances entre le transducteur de sonde (38) et les transducteurs de référence individuels (37a-d), le circuit de commande (30) étant adapté pour générer des données de position en trois dimensions du transducteur de sonde (38) relativement aux transducteurs de référence (37a-d) et l'agencement thérapeutique (22), sur la base des calculs TOF, et
le circuit de commande (30) étant adapté pour générer des informations de coordonnées locales du transducteur de sonde (38) relativement à l'agencement thérapeutique (22).

12. Système HIFU selon la revendication 11, dans lequel le circuit de commande (30) est adapté pour générer les données de position en trois dimensions avec un algorithme d'échelle multidimensionnelle (MDS).

13. Système HIFU selon la revendication 12, dans lequel le circuit de commande (30) est adapté pour générer les informations de coordonnées locales en utilisant une transformation de similarité Procrustéenne (PST).

14. Système HIFU selon la revendication 11 comprenant en outre un transducteur de pression disposé à l'extrémité distale de la sonde (36).

15. Système HIFU selon la revendication 11, dans lequel le transducteur de sonde (38) est un transducteur acoustique et de pression combiné.
